# EUROPEAN PATENT APPLICATION

(11) **EP 4 582 043 A2**
(43) Date of publication of application: **09.07.2025**
(21) Application number: 23859547.4
(22) Date of filing: 01.09.2023
(51) Int. Cl.: A61B 34/30, A61B 90/50, A61F 9/007, G02B 7/02, G10L 25/78, H04R 1/08, H04R 1/10, H04W 88/02

(54) **INTEGRATED MECHATRONIC SYSTEM FOR MANAGING OPHTHALMOLOGY SURGERIES UNDER THE FREE HANDS AND FEET CONCEPT THROUGH EXECUTION OF VOICE COMMANDS**

(30) Priority: 01.09.2022 US 202263403102 P; 15.03.2023 AR P230100649
(71) Applicant: Impel IP, LLC, Wilmington, DE 19801 (US)
(72) Inventor: BONETTO, Fabián José, San Carlos de Bariloche, Rio Negro, 265 (AR); SARAVIA, Mario Joaquín, Ciudad Autónoma de Buenos Aires, 1061 (AR); GÓNGORA, Nicolás Oscar, María Juana, Santa Fe, 2445 (AR)
(74) Representative: Durán-Corretjer, S.L.P.
(86) International application number: PCT/IB2023/000512
(87) International publication number: WO 2024/047397

(57) **Abstract**

Integrated system, attachable to a surgical microscope using magnets, capable of interpreting voice commands and processing images and videos in real time, to then execute functions on surgical instruments, such as optical and peripheral equipment usable in general surgery or, in particular, in ophthalmological surgeries, such as anterior and posterior segment, ocular plastics, orbit, oncological and other surgeries. The system automates the task of the surgeon and assistants when variations are needed during surgery, such as: visualization variations in focus (autofocus) or field adjustment systems (autofield), or performance of the surgical equipment, such as digital interventions in the foot and/or touch controls, interventions in existing knobs and/or levers for lighting control, and the like. The system includes magnetic quick couplings, sensor parts such as microphone, microprocessing center for image and video processing, and actuating parts such as stepper motors, direct current motors with gearboxes, servomotors, so that it allows replacing operations of surgeons and assistants, reducing the need for personnel in the operating room to execute actions, simplifying the task, reducing time and deviations or distractions from the sensitive manipulations performed by the surgeon.

## Description

### Field of the invention

The present invention refers to an innovative mechatronic system that optimizes time and reduces surgeon's avoidable movements during procedures performed using surgical microscopes. It is an accessory system for any surgical microscope, which is why its field of action covers the medical practice of microsurgery in general and, in particular, the field of ophthalmology. Preferably, the system, which has a set of quick couplers using magnets for quick assembly on the microscope including magnetic quick coupling lenses, integrates voice command functions, video image processing, automation that corrects focus and/or field of view with lens positioning in 3D space following the movement of the eye, and can electronically execute mechanical and non-mechanical commands, for example digital commands, on peripheral devices used in microsurgery in general and, particularly, in ophthalmological surgical procedures. In particular, it can be applied during vitreoretinal surgical procedures, i.e. ocular fundus surgery, to be able to maintain a dynamic and correctly focused visualization throughout the surgery, without the need for the surgeon to use his/her hands and/or feet. The surgeon does not need to take his/her hands off the microsurgery performed by him/her to obtain and maintain proper focus. This system is also applicable to other areas of microsurgery, where this accessory can hold and mobilize instruments for diagnostic-therapeutic purposes, using voice commands.

### Background of Previous Art

Surgery in general, and ophthalmological surgery in particular, uses complex pieces of equipment the functions of which must be configured while surgery occurs, mainly in procedures that use surgical microscopes. This task is carried out by both the surgeon and the auxiliary team.

In general, orders and actions to configure medical pieces of equipment, particularly surgical microscopes, are transmitted mechanically or electromechanically through the use of a keyboard, touch screens, switches, knobs or manual adjustment wheels or peripherals like pedals.

Therefore, it is necessary to have an integrated mechatronic system, attachable to a surgical microscope using magnets, for the management of surgeries in general, and ophthalmological surgeries in particular, under the concept of free hands and feet through the execution of voice commands, which allows replacing operations by surgeons and assistants, reducing the need for personnel in the operating room to execute actions, simplifying the task, reducing time and deviations or distractions from the sensitive manipulations performed by the surgeon.

### Summary of the Invention

The present invention consists of an integrated system, which is quickly coupled to a surgical microscope using magnets, which is capable of interpreting voice commands and processing images and videos in real time, to then execute functions in optical and peripheral equipment usable in microsurgery in general and in ophthalmology in particular, mainly in ocular fundus surgical procedures, as well as in other ophthalmological surgeries such as anterior, posterior segment, ocular plastics, orbit, oncological and other surgeries.

This system, which can be mounted and adapted to any surgical microscope using magnetic couplings, facilitates the task of the surgeon and assistants when variations are needed during surgery, such as: visualization variations in focusing systems (autofocus) or field adjustment (autofield), or performance of the surgical equipment, avoiding interventions such as the use of commands on the foot and/or touch controls, interventions on existing knobs or levers to control lighting and the like.

These actions are common in both general and ophthalmological microsurgery equipment and when operated using hands and/or feet by the surgeon and/or by assistants without a system like the one presented herein, they necessarily interrupt the specific and critical tasks that must be performed in surgery.

The quick coupling system based on the concept of magnets has the feature of a gold plating process, which makes it optimal for surgical areas. The gold is deposited on the magnets in the form of a thin layer about 5 nanometers thick, which allows a biocompatibility that the magnet as such does not possess. The gold-coated magnets have been tested by separating and joining the magnets a large number of times, which made it possible to verify that there is no appreciable deterioration in biocompatibility.

It should be noted that gold could be replaced by other precious metals such as platinum, palladium or precious metal alloys.

### Brief Description of the Figures

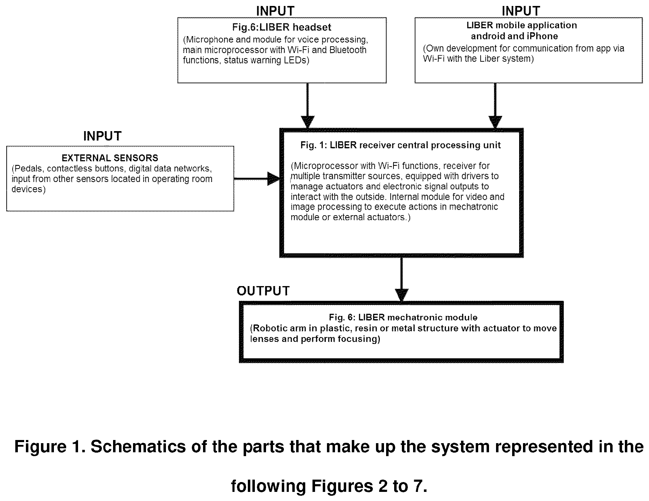

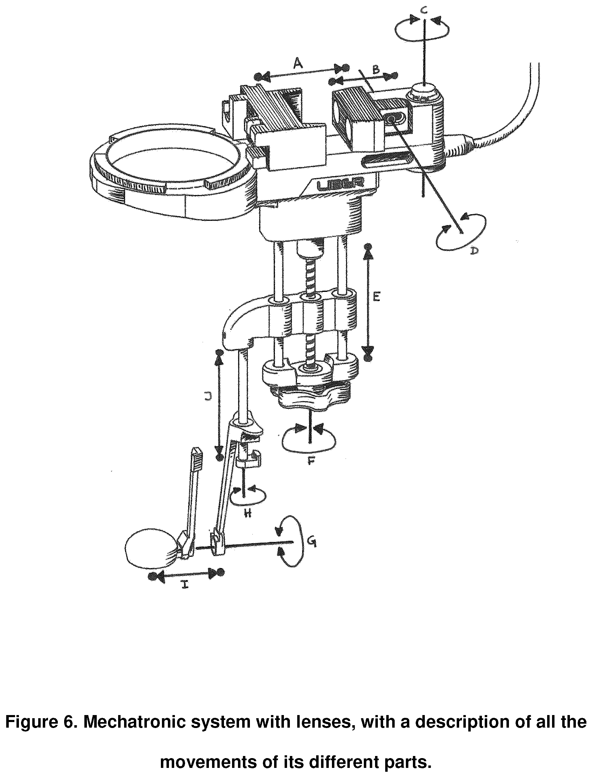

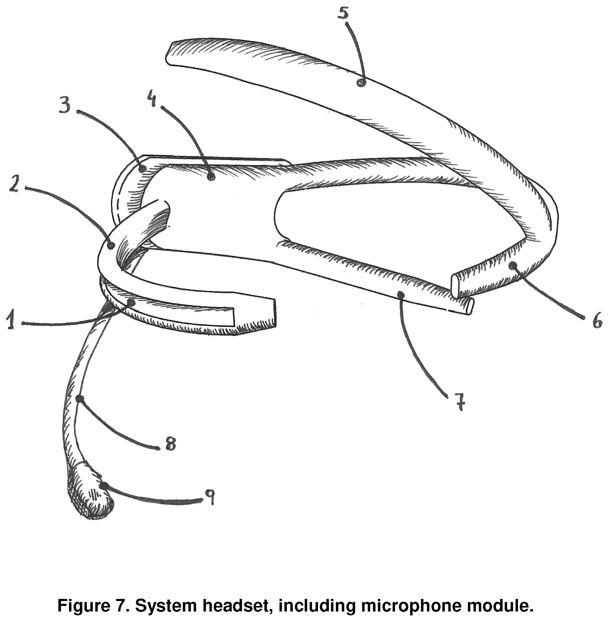

**Figure 1** represents a complete diagram of the parts that make up the electromechanical system called "Liber", which includes image and voice processing functions, with customized analog and serial digital outputs to execute actions in optical viewing and peripheral control systems used in this case in ophthalmological surgeries. The "headset" component (seen individually in Figure 6) is used interacting with Android or iPhone platforms. The voice processing system is configured from the mobile app. Likewise, for the iPhone platform, the headset can be optionally replaced by a product already developed by the Apple company, called "Airpods"; and in this case, these devices will be the ones that interact, sending commands (information) to the central processing unit. This way, each user will be able to use their own mobile device, iPhone or Android, in which they must download a specific application, developed to process voice commands and interact with peripherals, sending a command for the execution of orders.

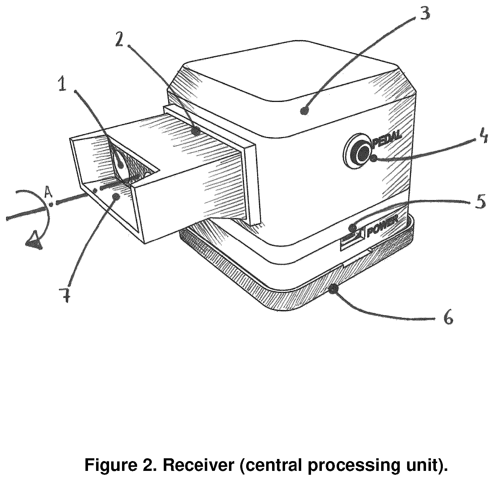

**Figure 2** shows the receiver, central processing unit, of the system, individually. Said module is powered with 5vdc from powerbanks or with a 220 Vac to 5 Vdc power supply. This module has a microcontroller with Wi-Fi functions that allows receiving orders from the headset by voice command. It can also receive optical, digital, analog or wireless signals from other peripherals and then process them and issue commands to execute actions in the mechatronic module.
   Part A of the image is the magnetic support socket of the mechatronic module when it is not used by the professional, which remains waiting in careful rest without contact with outside agents. According to the installation position of this module, the part is rotated so that the mechatronic module is suspended vertically when not in use. The parts of the receiver in figure 2 are:
   1- Pair of quick-coupling magnets between the receiver and the mechatronic module for rest when not used by the professional.
   2- Box with the magnets module.
   3- Electronic box.
   4- Input and output connectors of the mechatronic module, pedal, LCD.
   5- Power supply connector.
   6- Magnetic coupling base for quick installation of the receiver module.
   7- Support area of the mechatronic module.

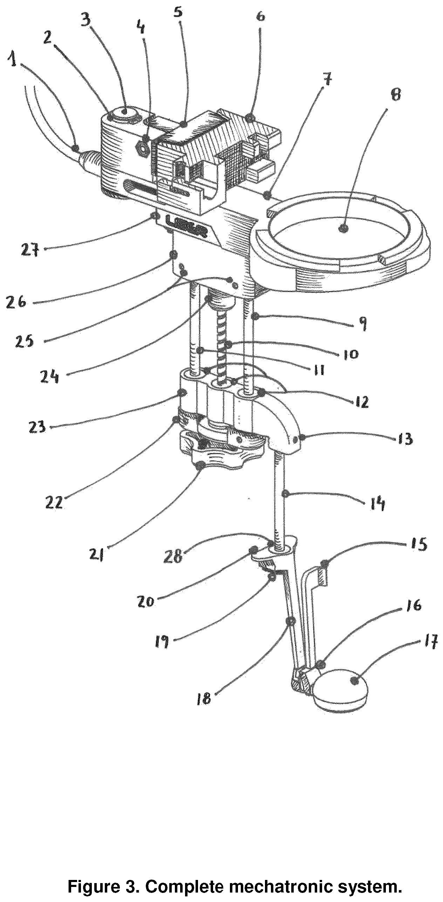

**Figure 3** shows a complete diagram of the mechatronic module that is attached to the surgical microscope that allows holding lenses and making movements between them. These commands are generated in the Liber receiver module and sent to the mechatronic module. The parts are described below:
   1- Cable and rubber finishing.
   2- Seeger bolt ring.
   3- 360° vertical pivot bolt.
   4- Nut insert for calibration adjustment to each microscope.
   5- Module with quick-coupling magnets on Liber side.
   6- Module having quick coupling to microscopes with magnets for connection to Liber.
   7- Upper lens support arm.
   8- Upper lens.
   9- Guide for automation.
   10- Worm screw for automation.
   11- Guide for automation.
   12- Bushings for guide and worm screw.
   13- Grub screw
   14- Guide to release lower lens.
   15- Lower lens support handle.
   16- Lower lens support.
   17- Lower lens.
   18- Liber connection extension to lower lens.
   19- Extension stop to lower lens.
   20- Lower lens extension module handle.
   21- Knob.
   22- Automation guide stop.
   23- Main automation arm.
   24- Motor and worm screw connection bushing.
   25- Automatism guides grub screws.
   26- Engine cover.
   27- Liber Main body.
   28- Lower lens extension module guide bushing.

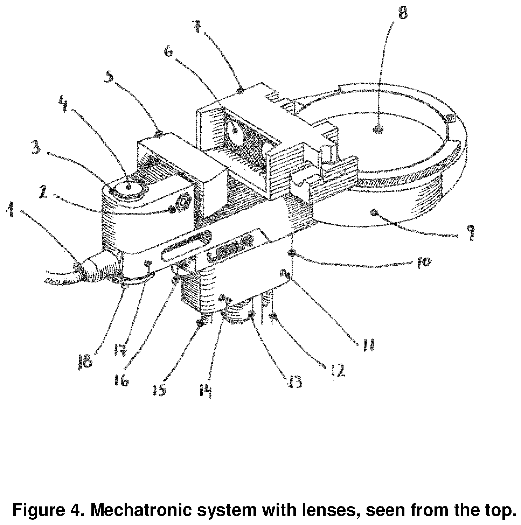

**Figure 4** represents the upper part of the mechatronic system, observing the parts detailed below:
   1- Cable and rubber finishing.
   2- Nut insert for calibration adjustment to each microscope.
   3- Seeger bolt ring.
   4- 360° vertical pivot bolt.
   5- Module with quick-coupling magnets on Liber side.
   6- Pair of quick-coupling magnets on the module side for mounting on microscopes.
   7- Module box for mounting microscopes.
   8- Upper lens.
   9- Upper lens support ring.
   10- Engine cover.
   11- Automatism guides grub screws.
   12- Guide for automation.
   13- Engine and worm screw connection bushing.
   14- Automatism guides grub screws.
   15- Guide for automation.
   16- Screw from engine cover to main body.
   17- Main body and upper lens support.
   18- 360° vertical pivot bolt.

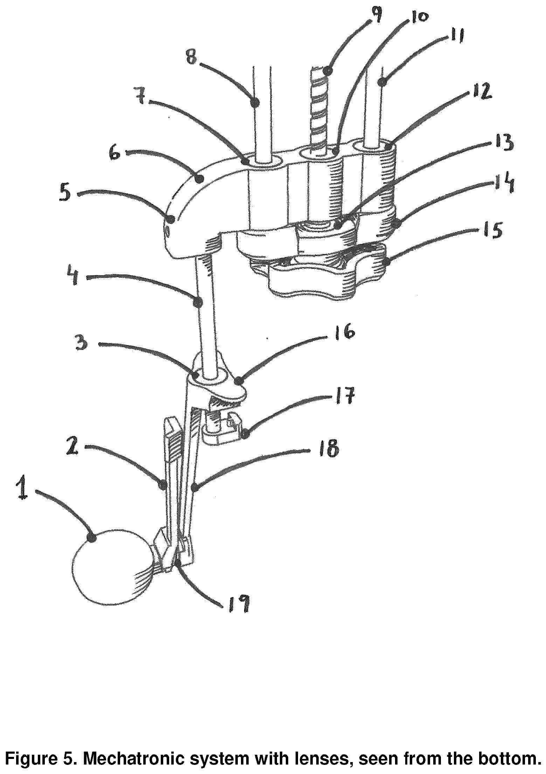

**Figure 5** the mechatronic system is observed with lenses, from another position, highlighting the following parts:
   1- Lower lens.
   2- Lower lens support.
   3- Extension module guide bushing to lower lens.
   4- Guide for lower lens release.
   5- Captive fastener for the lower lens release guide.
   6- Main automation arm.
   7- Bushing for automation guide.
   8- Guide for automation.
   9- Worm screw for automation.
   10- Worm screw bushing.
   11- Guide for automation.
   12- Bushing for automation guide.
   13- Worm screw lower main bearing.
   14- Automation guide stop.
   15- Knob.
   16- Lower lens extension module handle.
   17- Extension stop to lower lens.
   18- Liber connection extension to lower lens.
   19- Magnetic coupling between the lower lens support and the lower lens extension module.
**Figure 6** presents the mechatronic system with lenses, in such a way that all the movements of the pieces can be observed and described as stated below:
   A- Quick coupling movement between magnetic module for mounting on microscopes and magnet module on the Liber side.
   B- Linear movement in adjustment screw for Liber installation to microscopes.
   C- Liber vertical pivot 360° rotation movement.
   D- Rotation movement in adjusting screw for Liber installation to microscopes
   E- Liber automation vertical movement.
   F- Knob, worm and motor rotation movement.
   G- Lower lens rotation movement.
   H- Rotation movement of extension module to lower lens in the release guide.
   I- Coupling movement between the lower lens support lens and the lower lens extension module.
   J- Vertical release movement to avoid hitting patients.
Finally, **Figure 7** represents the "Headset" of the system, which consists of:
   1- Front lighting arm.
   2- Front fastening arm.
   3- Electronic module.
   4- Main fastening arm.
   5- Upper fastening arm.
   6- Lateral fastening arm.
   7- Rear fastening arm.
   8- Arm to microphone module.
   9- Microphone module and light warnings.

### Detailed description of the invention

In order to overcome the problems of the prior art, the present invention proposes an integrated mechatronic system to manage surgical procedures in general and ophthalmological surgeries in particular, under the "free hands and feet" concept, by executing voice commands, which comprises four parts: (a) headset-voice command, (b) mechatronic module, (c) receiver-central processing unit and (d) video and image analyzer. They are described below:
**a. Headset-voice command:** is a voice processing module comprising an input for a voice sensor, and an integrated memory for:
   saving different commands, saving different voices,
   being able to differentiate users,
   configuring commands according to each professional,
   performing functions without using hands or feet, and
   displaying automatic reports on display peripherals or audio reports (voice or sound).
**b. Mechatronic module:** a robotic system to align the axis of the optical/camera complex by sensing the real-time position of the visual axis by means of a tracking system, comprising:
   an automated robotic arm that modifies its position in space,
   lens and mirror modules for adjustment of optical directions in line with the direction of the visual axis, and
   an image processing module to control the automation that moves microscopes, cameras or devices with image and/or video sensors;
   an internal memory for sequential and chronological recording of commands used.
**c. Receiver-central processing unit:** outputs programmed to adapt to various peripherals and electronic equipment, said outputs selected from:
   digital outputs, on/off, pulse width;
   analog outputs;
   serial outputs, or specific ones adapted to interact with existing products on the market; and
   comprising an adaptable internal software arrangement to achieve feedback and execute actions in the software and hardware of the different commercial devices (API); and
   a universal conventional power supply.
**d. The image and video processing module,** which includes an input for video processing, and has the function of executing the following functions in real time:
   focusing through adjustments of optical and/or mechanical systems,
   adjusting the field through optical, mechanical or digital systems with command center functions, and
   comprising an adaptable internal software arrangement to achieve feedback and execute actions in software and hardware of different commercial devices (API); and
   a universal conventional power supply.

In another preferred embodiment, the integrated mechatronic system for managing surgical procedures in general and ophthalmological surgeries in particular, under the free hands and feet concept, through the execution of voice commands, is portable, and independent, coupling to the surgical microscope through a system of gold-coated magnets.

Preferably, the voice recognition system comprises a piezoelectric microphone sensing part, a voice recognition module, preferably Elechouse v3 with 16M-Bit serial flash memory to store voice audios in memory for later recognition. Also preferably, sending data from this module to the processing center is possible via cable and 3.5 mm stereo plug or wirelessly through the use of the esp8266 or esp32 chip. When the wireless method is used, the system is developed in the format of headsets with the capability to process voice and send it wirelessly to the central module. In order to provide warnings and indications, LEDs or a 2.44 cm (0.96") OLED screen with 128 x 64 pixels are incorporated into the headsets.

Preferably, an optical adjustment of the lens system is achieved by means of actuators, for example, adjusting the vertical position of the lower lens as well as its roll and tilt movement is carried out with stepper motors as direct current motors with gear boxes. Preferably, the stepper motor models used are 28byj, Y15-50A, or 4-wire and 2-phase replacements with control electronics developed with the UNL2003 driver. The direct current motors with gearboxes are model N20 and have different speeds according to their location in the equipment, the latter being controlled by the L293 driver.

A worm screw responsible for vertical movement provides a movement of 3 mm per turn and is made, for example, of bronze. The gimbal pulley on the lower lens allows complete roll and tilt movements of up to 120 degrees. In microscope equipment, that may allow so due to its morphology, the tilt movement will be carried out from its coupling base through the use of stepper or direct current motors mentioned above.

Preferably, the central processing unit comprises an atmega328p or atmega328au microprocessor equipped with a set of components that allow perfect performance in noisy environments. Likewise, also preferably, the use of an esp8266 and esp32 microprocessor is also added for models that require wireless WIFI or Bluetooth connections. Through these wireless or wired connections, this central unit will be able to receive information from the voice recognition module and will be able to send signals to execute actions on the actuators. To cover the need for warnings and indications, LEDs or a 2.44 cm (0.96") OLED screen with 128 x 64 pixels are incorporated into the central module.

Preferably, the video processing unit comprises an HDMI bridge board and a processing board selected from Rockchip RK3399, ARM-Cortex and STM32, depending on the processing power required in image processing. Likewise, preferably, the unit also allows the connection of FullHD quality video cameras up to 15 MegaPixels in those microscopes that do not have cameras installed. Preferably, the technique used for processing the autofocus function is pixel gradient analysis. Similarly, the technique used for field adjustment processing (autofield) is the contrast analysis between the peripheral black zone and the illuminated zone. Finally, the technique used to control brightness in the field of view and avoid light toxicity is the control of image contrast and brightness.

Also preferably, the central actuator for peripherals and surgical equipment of the integrated mechatronic system for managing ophthalmological surgeries according to the present invention, allows the central unit to execute actions towards peripherals installed digitally, wired or wirelessly through the interactions achieved from the voice command module and image processing. In this way, the central module can move knobs, levers, keys and/or buttons existing in the system during surgeries using the aforementioned actuators or incorporating others of greater power such as linear pistons from 5 V direct current to 12 V direct current, with movement capacity up to 500 kg.

It can also interact in a wired manner and send HIGH, LOW digital pulses, analog signals, DAC, pulse width or specific serial pulses to be able to connect to existing electronic equipment under specific protocols. For example, in a preferred embodiment, for the central unit to automate foot controls, a wireless module is wired or installed to send digital signals into the controls. In this way, the use of feet by surgeons and/or assistants is eliminated. In another example of yet another preferred embodiment, for the control of diaphragms to reduce light toxicity in the eyes during operations, their aperture is adjusted using the video information processed in the central module and an actuator is installed to perform the movement

In another preferred embodiment, the Headset system is replaced by Airpods, for example the noise-canceling model, and the user's own iPhone. This set of Airpods + iPhone will be responsible for receiving voice commands and transmitting them to the receiving center, replacing the Headset system. For its operation, the iPhone's user will download a specific application, developed to be able to interact with this integrated mechatronic system.

## Claims

1. An integrated mechatronic system, attachable to a surgical microscope using magnets, for the management of surgeries in general, and ophthalmological surgeries in particular, under the free hands and feet concept through the execution of voice commands, which is in charge of performing movements of structures that can hold different types of surgical instruments, such as lenses attached to devices and contactless viewing cameras, wherein said system consists of:
a central processing module comprising a microprocessor, a driver for the movement of actuators installed in the lens system and a voice command module capable of working without mechanical intervention,
wherein the movement of the system that can hold lenses to facilitate focusing and defocusing is vertical from the camera towards the patient and responds through voice commands received by the medical staff; and
wherein a system that can hold surgical instruments such as lenses with actuators for optical adjustment is a system composed of lenses and actuators for performing movements that improve image quality on surgical display monitors.

2. The integrated mechatronic system, attachable to a surgical microscope using magnets, for the management of surgeries in general, and ophthalmological surgeries in particular, under the free hands and feet concept through the execution of voice commands, which is in charge of performing movements of structures that can hold different types of surgical instruments, such as lenses attached to devices and contactless viewing cameras, according to claim 1, wherein said system further consists of:
headsets that include a microphone, speakers, voice command processor and WIFI system for sending commands and receiving signals from the central processing unit, or that integrates "Airpods + iPhone", with its own application developed to receive voice commands, process them and connect via WIFI system to the central processing unit;
a central processing module that includes a microprocessor, WIFI system for communication with doctors' headsets, a driver to perform movements in the lens system, additional input for end stroke sensors or real-time control of PDM consumption (Power Distribution Module); and
a structure attachable by means of a system of gold-coated magnets, which can hold/incorporate surgical instruments, such as a lens system with actuators for optical adjustment, comprising a metal and/or plastic structure and 3D printing in resin, actuators and sensors, with the capability to make movements in different directions that can be used to perform focusing and movement functions.

3. The integrated mechatronic system, attachable to a surgical microscope using magnets, for the management of surgeries in general, and ophthalmological surgeries in particular, under the free hands and feet concept through the execution of voice commands, which is in charge of performing movements of structures that can hold different types of surgical instruments, such as lenses attached to devices and contactless viewing cameras, intervening in the peripherals and electronic devices existing in surgeries to automate them, according to claim 1, wherein said system further consists of:
headsets that include a microphone, speakers, voice command processor and WIFI system for sending commands and receiving signals from the central processing unit, or that integrates "Airpods + iPhone", with its own application developed to receive voice commands, process them and connect via WIFI system to the central processing unit;
a central processing module that includes a microprocessor, WIFI system for communication with doctors' headsets, a driver to perform movements in the lens system, additional input for end stroke sensors or real-time control of PDM consumption (Power Distribution Module), video or image processing to perform actions in real time, such as optical adjustments, activation of peripherals and activation of electronic devices used in operating rooms;
a structure attachable by means of a system of gold-coated magnets, which can hold/incorporate surgical instruments, such as a lens system with actuators for optical adjustment that comprises a metal and/or plastic structure and 3D printing in resin, actuators and sensors, with the ability to perform movements in different directions that can be used to perform focusing and movement functions; and
a central actuator for peripherals and surgical equipment that includes drivers and microprocessors programmed so that the central processing module can command other equipment installed in the operating room, thus automating levers, pedals, knobs, keys, or injecting digital signals to achieve an effective freehand in an ophthalmological operation.

4. The integrated mechatronic system, attachable to a surgical microscope using magnets, for the management of surgeries in general, and ophthalmological surgeries in particular, under the free hands and feet concept through the execution of voice commands, which is in charge of performing movements of structures that can hold different types of surgical instruments, such as lenses attached to devices and contactless viewing cameras in ophthalmological surgery microscopes, intervening in the peripherals and electronic devices existing in surgeries to automate them, according to claim 1, wherein said system further consists of:
a wireless voice recognition system capable of manipulating multi-peripherals and electronic equipment used in medical surgery rooms, under the hands-free concept, comprising:
headsets that include a microphone, speakers, voice command processor and WIFI system to send commands and receive signals from the central processing unit, or that integrate "Airpods + iPhone", with its own application developed to receive voice commands, process them and connect via WIFI system to the central processing unit and
a central processing module that includes a microprocessor, WIFI system for communication with doctors' headsets, a driver to perform movements in external actuators, additional input for end of stroke sensors or real-time control of PDM (Power Distribution Module) consumption video or image processing to perform selected real-time actions for optical adjustments, actuation of peripherals and electronic devices used in operating rooms, wherein these actions are carried out through inputs and outputs programmed to interact with the outside world through electronic and mechanic devices installed in operating rooms.

5. The integrated mechatronic system, attachable to a surgical microscope using magnets, for the management of surgeries in general, and ophthalmological surgeries in particular, under the free hands and feet concept through the execution of voice commands, which is in charge of performing movements of structures that can hold different types of surgical instruments, such as lenses attached to devices and contactless viewing cameras in ophthalmological surgery microscopes, intervening in the peripherals and electronic devices existing in surgeries to automate them, according to claim 1, wherein said system further consists of:
headsets that include a microphone, speakers, screen, LED warnings, voice recognition module and microprocessor with WIFI function to send voice commands wirelessly to the central processing module, or that integrate "Airpods + iPhone", with its own application developed to receive voice commands, process them and connect via WIFI system to the central processing unit;
a microprocessor central processing module with WIFI function to receive voice commands from the headset;
drivers to manipulate actuators attached to medical equipment that must be automated;
inputs of end stroke and position sensors, and potentiometers, from the outside;
connectors to be linked by digital signals to various electronic equipment to operate them;
a video processing module with or without a camera for selected real-time adjustments of autofocus, autofield and light toxicity;
an actuator module to automate optical adjustment in lenses, diaphragm adjustments and movements of knobs or pedals existing in operating rooms; and
voice command function to reduce mechanical movements of medical personnel in surgeries.
